# EUROPEAN PATENT APPLICATION

(11) **EP 2 159 195 A1**
(43) Date of publication of application: **03.03.2010**
(21) Application number: 08718417.2
(22) Date of filing: 15.01.2008
(51) Int. Cl.: C01B 31/20, C12S 5/00

(54) **ACCELERATED METHOD FOR CONVERTING CARBON DIOXIDE INTO ENERGY**

(30) Priority: 16.01.2007 ES 200700141
(71) Applicant: Stroïazzo-Mougin, Bernard A. J., 03560 El Campello (Alicante) (ES)
(72) Inventor: Stroïazzo-Mougin, Bernard A. J., 03560 El Campello (Alicante) (ES)
(74) Representative: Elzaburu Marquez, Alberto
(86) International application number: PCT/ES2008/000016
(87) International publication number: WO 2008/087238

(57) **Abstract**

The present invention relates to a process for the energy conversion of carbon dioxide, comprising the steps of culturing phytoplankton in electromagnetic bioaccelerators, producing oxygen and biomass made up of lipids, hydrocarbons and sugars from the previous step, oxidizing the hydrocarbons produced in the previous step to generate carbon dioxide and NOx and collecting the carbon dioxide and NOx from the previous step until the cultures of the first step.

## Description

### Technical Field of the Invention

The present invention relates to the field of the use of renewable energies and to the field of obtaining electric and thermal energy by means of the use of electromagnetic bioaccelerators and the mass culture of phytoplankton and zooplankton type organisms, the phytoplankton organisms usually belonging to the following taxonomic families: Chlorophyceae, Bacillariophyceae, Dinophyceae, Cryptophyceae, Chrysophyceae, Haptophyceae, Prasinophyceae, Raphidophyceae, Eustigmatophyceae, and the zooplankton organisms usually belonging to the Copepod, Thaliacea, Cladocera, Rotifera and Decapod families ... generally the taxonomic families comprising species of the chromophyte division, all of them characterized by being flagellated or nonflagellated single-celled organisms and with a strictly planktonic (holoplanktonic) life phase, or at least one of its phases being planktonic (meroplanktonic). The species of the group of phytoplankton organisms the use of which is related to the present invention are, in a non-limiting manner: *Dunaliella salina, Tetraselmis sp, Isochrysis galbana, Pavlova lutheri, Rhodomonas salina, Phaeodactylum tricornutum, Thalassiosira weissflogii* and *Chaetoceros socialis*.

The present invention specifically relates to a process for using the CO₂ emitted by the combustion of the biofuel obtained from said microorganisms (which have been generated by means of photosynthesis and cell mitosis,) and using the generated CO₂ to reproduce the microorganisms. The massive capture of gases with a greenhouse effect and accordingly a global warming effect, especially carbon dioxide, is thus achieved.

### State of the Art

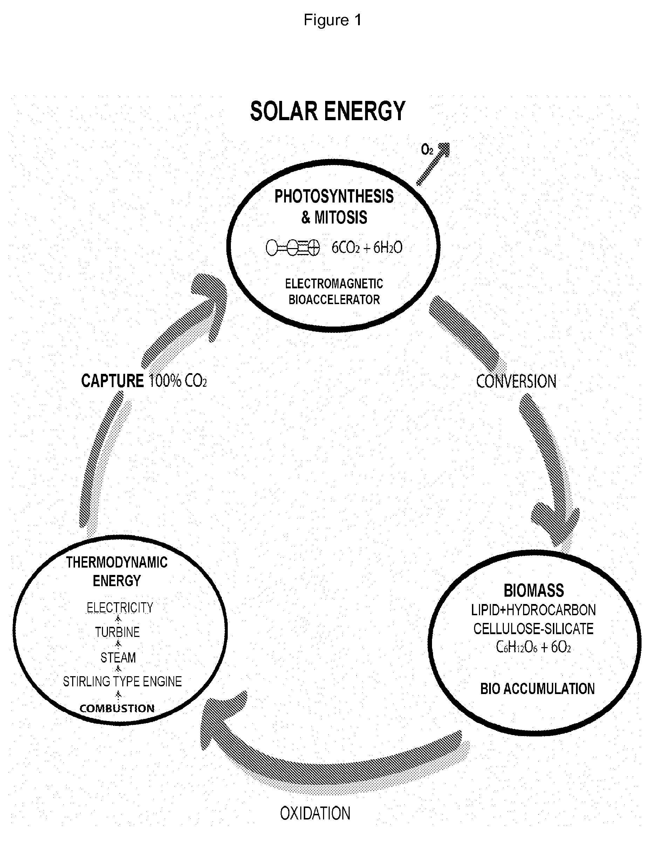

Global warming is the theory which states that there is an increase in the average temperature of the Earth's atmosphere and of the oceans due to the greenhouse effect caused by the emission of carbon dioxide and other gases. In this same sense, the temperature has increased since the end of the 19^{th} century when a period of about 400 years known as the "minor glaciation" period ended, and it is estimated that this warming is largely due to human activity, which has increased during recent decades. The theory furthermore predicts that temperatures will continue to rise in the future if the emission of greenhouse gases continues (Figure 1).

The obligation for the economic zones to comply with the objectives imposed by the Kyoto protocol on the reduction of CO₂/SO₂ emissions and the emission of other gases causing the so-called greenhouse effect is forcing countries to search for alternative and renewable fuels to prevent possible penal taxes.

Although the production of solar and wind energy is increasing in some regions, these technologies are very expensive and are not viable in all climatic areas. In these conditions, biofuels have an important role as substitutes of fossil fuels, especially in transport and heating applications.

The production costs of biofuels from plants, such as palm and rapeseed oil, have always been a reason for concern. Taking into account the low oil production indexes per hectare, enormous amounts of resources would be needed to reach commercial production. Land and water are two limited resources and it is preferable to use them to produce food products, which are furthermore more profitable for farmers. Intensive fertilization is furthermore an enormous form of land and water pollution. Extensive single crop farming is also one of the main enemies of biodiversity.

A study conducted by the University of California-Berkeley, Natural Resources Research Vol 14 No. 1 March 2005 pp. 65-72, shows that a terrestrial plant such as sunflower uses up more energy than it produces; for example to produce 1,000 Kg of sunflower fuel having a power of 9,000,000 Kcal, 19,000,000 Kcal of energy must be used, which corresponds to a CO₂ emission exceeding a fossil fuel emission; for example a 135 hp car traveling 100 Km emits a value of 20 Kg of CO₂ with a fossil fuel. When a sunflower-based fuel is used, the total combined emission would be 36 Kg of CO₂; however when the fuel is based on phytoplankton, after the recovery of the CO₂ from a thermal power plant for example, the balance is 10 Kg of CO₂ emitted into the atmosphere due to the collection of the same car having the same horsepower on the 100 km trip; the reason is that the CO₂ captured from the factory has generated a power of 100 Kw and has been captured by the algae which, at that time, leave a balance of 0; however, since algae produce the biofuel that will drive the car 100 Km, this biofuel is going to emit the same as fossil fuels, about 20 Kg, but the total balance is 200 Kw per 20 Kg and therefore the net result will be 10 Kg. However, the present invention describes an accelerated process in which since a part is recovered before producing the fuels, i.e. part of the body of cells for making inert products such as silicates, cellulose, etc. is recovered. This part allows reducing the CO₂ captured for conversion by 30%, and therefore the net result is 4 Kg of emission of CO₂ in contrast with the 10 Kg previously generated.

Finally, total CO₂ recycling is achieved by means of the present invention and therefore the balance is 0 given that all the generated CO₂ returns to the cultures in order to nourish the phytoplankton and thus again generate biomass. The need to generate systems exploiting the use of phytoplankton to generate clean energy and which do not negatively affect the Earth is therefore evident.

In view of the foregoing, phytoplankton represents a viable solution to the previously discussed problem given that about 50% of the dry mass of single-celled organisms is generally biofuel. In addition, the annual production per hectare of biofuel from phytoplankton is 40 times higher than with the second most cost-effective product, palm oil. A drawback is that the production of phytoplankton oil requires covering vast stretches of land with rather shallow water, as well as introducing large amounts of CO₂, an essential element for phytoplankton to produce oil. Natural production systems, such as phytoplankton ponds, have a relatively low cost but the harvesting process is very laborious and therefore expensive. In addition, phytoplankton culturing is carried out in open systems, making it vulnerable to pollution and to problems for cultures, which may lead to total production loss. In this same sense, an advantage of the process described in the present invention is that the process object of the present patent is carried out by means of electromagnetic bioaccelerators, which are closed systems, and in conditions such that the culture is not contaminated by bacteria, fungi,... because in addition to being closed, the culture is enriched by means of nutrients incorporating fungicides and antibiotics.

An electromagnetic bioaccelerator is understood to be a system using natural elements, such as photosynthesis, mitosis and electromagnetism, such that the molecular exchange at the phytoplankton level useful as an energy capture, transport and transformation vehicle is accelerated. In summary, it is a system which accelerates the natural process of photosynthesis and transformation of electromagnetic energy into biomass.

Until now no similar processes incorporating electromagnetic bioaccelerators have been described which further incorporate the advantages of being a closed system with a large volume and large diameters, which works continuously, which allows obtaining large amounts of biofuels or byproducts such as naphthas, glycerin, silicon-derived compounds, such as ferrosilicates, which may further obtain thermal and electric energy that does not contaminate given that all the possible residues, such as carbon dioxide, are recirculated in the system to be used as a nutrient for the phytoplankton, or which recirculates the used water as part of the culture medium so it can be reused...

Due to the ability of the electromagnetic bioaccelerator to accelerate phytoplankton and zooplankton reproduction by means of mitosis and its ability to accelerate photosynthesis, the present accelerated system for the energy conversion of carbon dioxide allows reaching very high production rates can be obtained that are almost equivalent to the energetic power of the fossil hydrocarbons. Furthermore, due to the design of the electromagnetic bioaccelerators as a constitutional part of the present process, it has the ability to recreate an environment that is similar to the sea (light, temperature and pressure) at a depth in which this phytoplankton is cultured and developed naturally. An essential feature of the present invention is that the electromagnetic bioaccelerator system regulates the phytoplankton and zooplankton culture conditions, such as temperature, pressure and light. Thermal regulation of the system is thus made easier, which in turn makes it easier to control phytoplankton and zooplankton populations being cultured, and reducing the energy costs necessary for maintaining the homoeothermic conditions in the culturing system. And as a second feature, it assures the availability of water with no limitation and high infrastructure costs of any kind.

Another advantage of the process object of the present invention is that it works with an electric field and a magnetic field which are present in the electromagnetic bioaccelerator, the ultimate purpose of which is to make phytoplankton production be high and to affect the electron exchange comprised in photosynthesis.

Therefore the present invention describes a novel system including all these features and allowing wide versatility and being very environmental-friendly.

In addition, there are currently methods or processes which make use of microalgae, as in the case of patent application WO 03/094598 A1 entitled "Photobioreactor and process for biomass production and mitigation of pollutants in flue gases", describing a generic photobioreactor model mainly focused on decontaminating COx, SOx and NOx type gases. It is basically a system working in a discontinuous manner (distinguishing between day/night photoperiod) and is open, its liquid medium not being axenic. It does not control nitrogen and carbon dioxide concentrations for the purpose of increasing biofuel production. It is not designed to work with monospecific or monoclonal algae strains. Its design does not contemplate biofuel production as the main objective, rather it is focused on gas purification. In addition, in relation to the photosynthetic organisms it refers to, it does not demand conditions disabling the system and it has no controlled recirculation because the transport is done by a turbulent flow of bubbles.

Compared to the present invention object of the patent, a completely novel system is set forth which is based, in contrast, on the following features:
- It is completely closed.
- It is completely axenic.
- It works continuously.
- It works with monospecific and monoclonal strains.
- It accepts mixed autotroph-autotroph, autotroph-heterotroph, facultative heterotroph-facultative heterotroph cultures.
- It does not accept just any photosynthetic organism, but rather it at least requires that they are not organisms forming biofouling on the inner surface of the electromagnetic bioaccelerator.
- It accepts facultative heterotrophs
- It requires that the phytoplankton and zooplankton species do not form colonies.
- It requires that the phytoplankton and zooplankton species do not generate exo-mucilage.
- It requires that the cultured species contains at least 5% of fatty acids and at least 5% of hydrocarbons.
- It enhances the use of nonflagellated and floating phytoplankton and zooplankton species.
- It does not accept just any type of liquids as culture medium, it focuses on freshwater, brackish water and seawater.
- Its main objective is to obtain metabolic synthesis compounds with energetic properties or with pre-energetic properties essentially aimed at obtaining biofuels.
- It uses the generation of biomass for the development of biofuels and other non-contaminating byproducts given that the CO₂ and NOx that they generate are reused by the bioaccelerators to restart the process described in the present invention.

### Description

The present invention relates to an accelerated process for the energy conversion of carbon dioxide, Figure 1, which consists of the following steps:
In a first step, the culture of phytoplankton is carried out, which phytoplankton is immersed in electromagnetic bioaccelerators, the main function of which is to accelerate photosynthesis and cell division by means of mitosis. The electromagnetic energy necessary for the culture of the phytoplankton comes from solar radiation and the supply of carbon is carried out by means of the CO₂ coming from combustion gases generated in the last step of the process described in the present invention, from the combustion of the biomass, or from the byproducts generated in the process and the exceeding Kcal of the combustion of the biomass will be used to maintain the temperature of the culture. As is known, any exchange of thermodynamic energy to electric or mechanical energy generates a 60% loss in thermal energy; however by means of the present process, since it is a closed cycle, part of the lost thermal energy is recovered to reheat the system and accelerate production.

Photosynthesis is understood to be the process by means of which plants, algae and some bacteria capture and use light energy (electromagnetic energy) to transform the inorganic matter of their external environment into organic matter that will be used for their growth and development. Photosynthesis is divided into two phases. The first phase occurs in thylakoids where light energy is captured and stored in two simple organic molecules (ATP and NADPH). The second phase takes place in the stromata and the two molecules produced in the preceding phase are used in atmospheric CO₂ assimilation to produce carbohydrates and, indirectly, the remaining organic molecules which make up living beings (amino acids, lipids, nucleotides, etc). In the first phase the light energy captured by the photosynthetic pigments attached to proteins and organized in the so-called "photosystems" causes water to be broken down, releasing electrons circulating through carrier molecules to reach a final acceptor (NADP⁺) that can mediate in the transformation of the atmospheric CO₂ (or dissolved in the water in aquatic systems) into organic matter. This light process is also coupled with the formation of molecules working as energy exchangers in cells (ATP). The formation of ATP is also necessary for CO₂ fixation.

6 CO₂ + 6 H₂O → C₆H₁₂O₆ + 6 O₂

In the second step of photosynthesis, the Calvin cycle is carried out in which cycle inorganic carbon dioxide molecules are converted into simple organic molecules from which the remaining biochemical compounds making up living beings will be formed. This process can therefore also be called carbon assimilation. It could therefore be verified that for a total of 6 fixed CO₂ molecules, the final stoichiometry of Calvin cycle can be summarized in the following equation:

6CO₂ + 12NADPH + 18 ATP → C₆H₁₂O₆P + 12NADP⁺ + 18ADP + 17 Pi

This would represent the formation of a 6 carbon atom sugar-phosphate molecule (hexose) from 6 CO₂ molecules.

The carbon chains forming the remaining molecules making up living beings (lipids, proteins, nucleic acids and others) will also be directly or indirectly formed from these sugars.

In order to carry out this first step it is necessary to control the temperature, to control the light intensity and the supply of nutrients. It must also be assured that the culture medium is axenic.

The conditions for being able to carry out this first step of the process are the following:
- constant temperature within the range of 20 to 25°C.
- solar light intensity from 200 to 900 watts/m².
- wavelengths within the range of 400 to 700 nm.
- artificial light intensity from 1 to 50 watts/ m².
- the photoperiods depending on the strain cultured will be within the following ranges:
   ○ 24:0 hours (light/dark).
   ○ 16:8 hours (light/dark).
   ○ 18:6 hours (light/dark).
   ○ 20:4 hours (light/dark).
   ○ 12:12 hours (light/dark).
- Salinity:
   ○ Salt water strains: 20‰-40‰.
   ○ Brackish water strains: 8‰-20‰.
   ○ Fresh water strains: 0,2‰-8‰.
- Concentration of phytoplankton in the culture medium from 30 million cells/ml to 500 million cells/ml.
- pH from 6.5 to 8.9.
- Pressure from 1 to 5 atmospheres.

The initial strains for the electromagnetic bioaccelerator inoculation will be maintained in microfiltered seawater using 0.45 micron cellulose acetate filters and subsequent 0.20 micron re-filtering, and finally sterilized using UV rays. The culture medium of the electromagnetic bioaccelerators will be kept sterile and axenic by means of antibiotics and fungicides.

The antibiotics added to the culture are a mixture of penicillin and streptomycin in a range of concentrations from 100 to 300 mg/l each, preferably in a range of concentrations from 150 to 250 mg/l and more preferably at a concentration of 200 mg/l for each of the components of the mixture.

The fungicides added to the culture are a mixture of griseofulvin and nystatin in a range of concentrations from 100 to 300 mg/l each, preferably in a range of concentrations from 150 to 250 mg/l and more preferably at a concentration of 200 mg/l for each of the components of the mixture.

The culture medium used is to sustain biomasses exceeding 100 million cells/ml, being a Guillard-type medium, according to the protocol described by Robert A., Andersen in the book Algal Culturing Techniques with ISBN 0-12-088426-7. Edited by Elsevier, 2005, pp. 507-511.

Said medium has been modified by doubling the nitrogen (N₂) concentrations for the purpose of exceeding cell concentrations exceeding 125 million cells/ml.

Therefore the second step of the present invention consists of the production of biomass (lipids, hydrocarbons and sugars) and oxygen coming from the mass culture of the phytoplankton present in the culture medium of the electromagnetic bioaccelerators. In addition, byproducts such as silicates or cellulose, which are a constituent part of the body of each of the cells of the culture medium, are produced. The methods used to extract the biomass from the culture medium are any of those methods described in the state of the art. However in order to separate the silicates and the cellulose, apolar solvents that are able to dissolve and extract these products, and which are described in the state of the art, were used for this purpose. In addition, the methods for breaking up the cells of the culture medium are, in a non-limiting sense, ultrasounds, polytron or grinding, microwaves and/or heating at 200°C.

All these products listed above which are the result of the capture and transformation of carbon dioxide, are indirectly carbon dioxide which is not returned to the atmosphere, but rather is used again by means of going from the last step of the present process to the first step of said process.

In the third step of the process, the products obtained in the previous step undergo an oxidation process by direct or indirect combustion in order to produce thermodynamic energy, which is used in vehicles or in electric power production plants. The residual products of this process are mainly NOx and carbon dioxide.

In the last step of the process, these residual products are again taken back to the electromagnetic bioaccelerators of the first step such that the cycle described in the present process is closed and these products are again used as nutrients for the culture medium in which the phytoplankton is present.

Therefore the thermal energy produced by all the carbon compounds is completely used. The transformation of the second step to the third step is by means of direct combustion after centrifugation and drying of the biomass. Once it is dry, it is injected into a furnace to use the gases in a heat exchanger which in turn produces steam that is sent to turbines. The remaining gases at the exchange output directly return to the electromagnetic bioaccelerator. On a smaller scale, the steam turbine can be replaced by a Stirling type engine using the high temperatures of the combustion chamber or furnace for the operation of this type of engine. The type of turbine used is any of those turbines described in the prior state of the art. A Stirling-turbine combined cycle fed by the combustion of the biomass generated in the second step of the present process can be used in intermediate conditions.

A Stirling type engine is understood to be one of those engines the main operating principle of which is the work done due to the expansion and contraction of a gas when it is forced to follow a cooling cycle in a cold reservoir, whereby it contracts, and a heating cycle in a hot reservoir, whereby it expands. In other words, the presence of a difference in temperatures between two reservoirs is necessary, and it is a heat engine. Its work cycle is formed by means of 2 isochoric transformations (heating and cooling at constant volume) and two isotherms (compression and expansion at constant temperature).

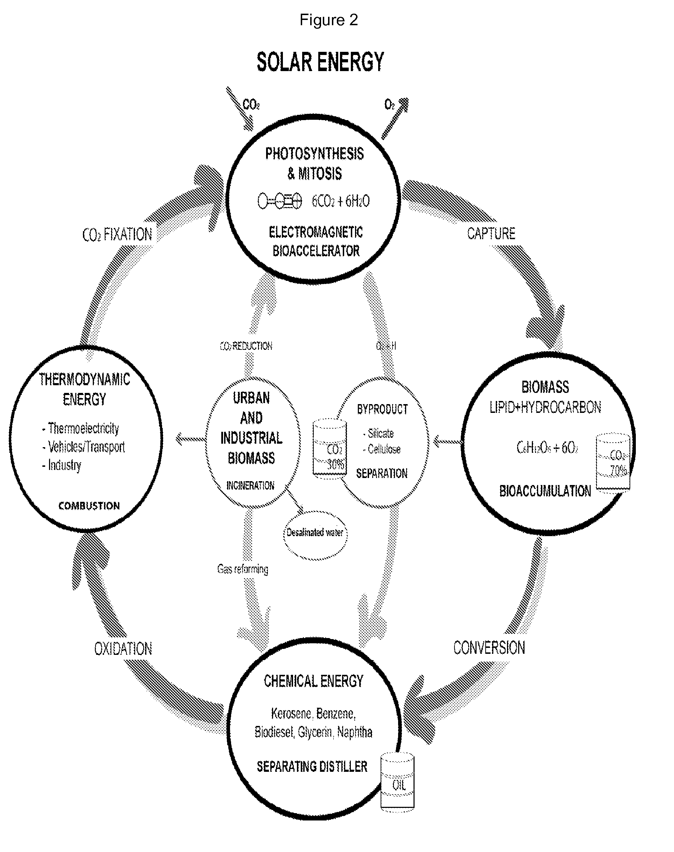

According to a preferred embodiment the accelerated cycle for the energy conversion of carbon dioxide would consist of 5 steps instead of 4 or, in other words, a fifth step is additionally incorporated to the process between steps 2 and 3 (Figure 2). In this new step a transformation process is carried out on the products obtained in the second step. The lipids are aimed at a chemical energy transformation process by means of transesterification. The hydrocarbons are distilled by means of catalytic hydrocracking, thus obtaining energy products such as kerosene, benzene, biodiesel, naphthas and others, such as glycerin. A molecular breakup is applied to the sugars in order to obtain ethanol, part of which will be used in the transesterification process carried out in the lipids.

Transesterification is understood to be the process carried out by means of the following chemical reaction:

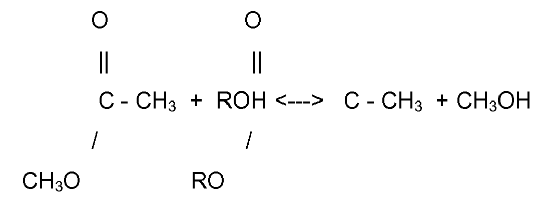

The third step would thus be avoided, depending on the needs of the system.

In the fourth step of the process, the hydrocarbons undergo an oxidation process by direct or indirect combustion to produce thermodynamic energy, which is used in vehicles or in electric power production plants. The residual products of this process are mainly NOx and carbon dioxide.

In the last step of the process, these residual products are taken back to the electromagnetic bioaccelerators of the first step such that the cycle described in the present process is closed and these products are again used as nutrients for the culture medium in which the phytoplankton is present.

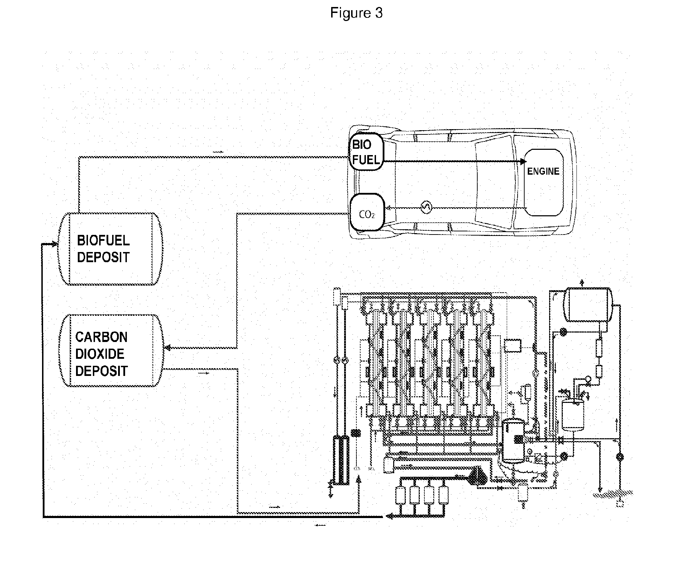

According to another preferred embodiment, the present process could be used to recover carbon dioxide emitted by car engines (Figure 3), which is captured at the exhaust pipe outlet in normal engine operating conditions. Then the gases are compressed and accumulated in a deposit independent from the vehicle, similar to a fuel deposit or tank. Then this deposit is emptied in service stations at the same time the vehicle is filled up with fuel. These carbon dioxide collection tanks or deposit are then reinjected in the electromagnetic bioaccelerators of an accelerated type energy production plant for the energy conversion of carbon dioxide while at the same time said plant produces the necessary fuel for the vehicle.

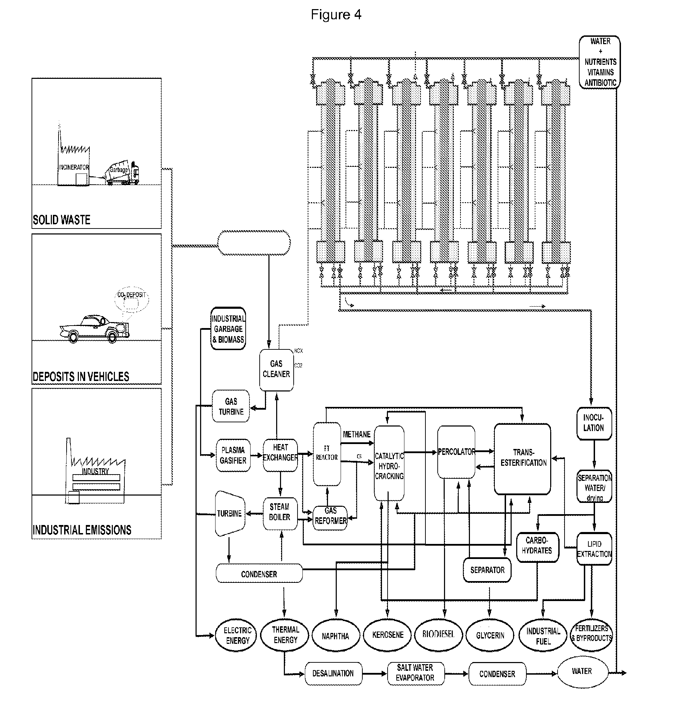

According to another preferred embodiment, the accelerated process for the energy conversion of carbon dioxide would be a constituent part of an incineration plant (Figure 4) such that a continuous source of carbon dioxide can be assured as a supply of nutrients to the electromagnetic bioaccelerators.

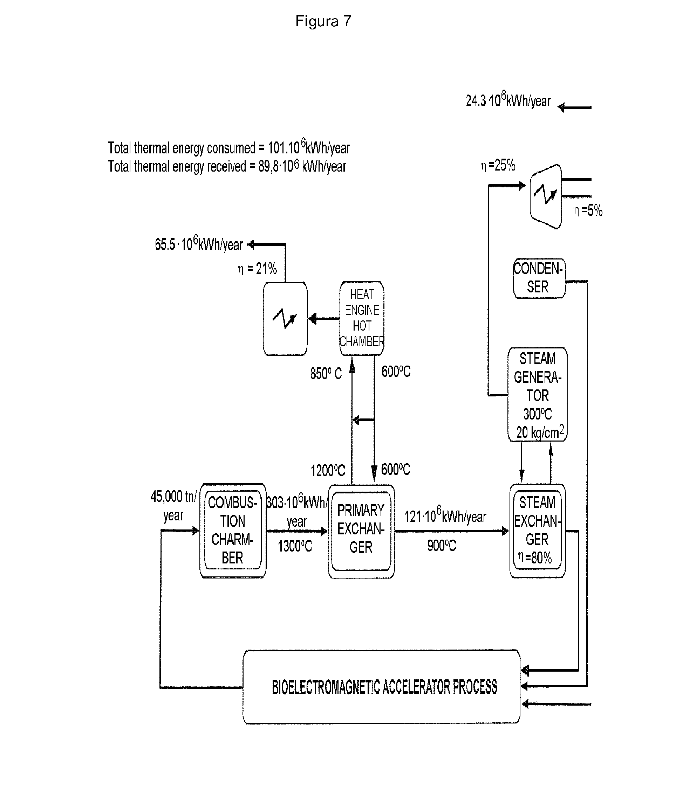

According to other preferred embodiments, the accelerated process for the energy conversion of carbon dioxide would work as shown in a 1h heat engine cycle bio-electric system (Figure 7), in which the calorific value of the biomass is used by a heat engine to generate electric energy and by a steam exchanger; this steam also allows producing electric energy through a steam turbine.

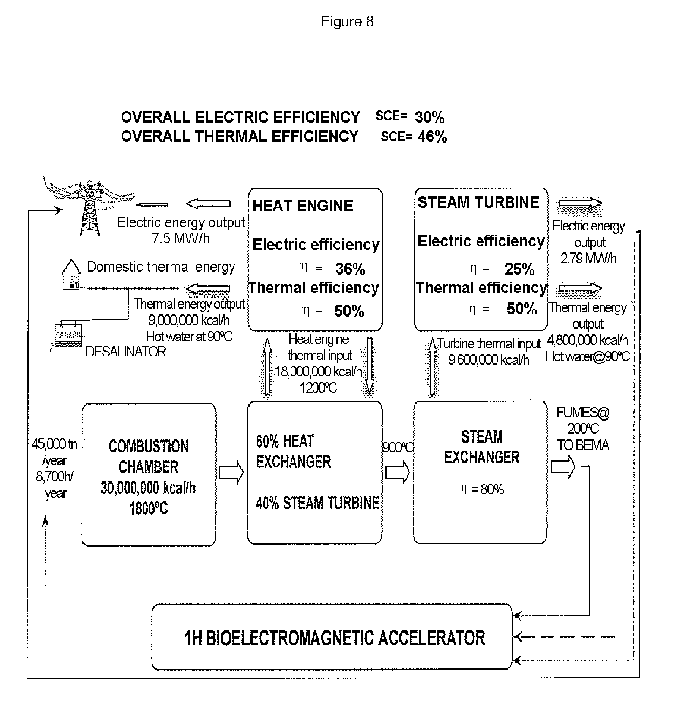

It would operate in this same sense like a 1h heat engine cycle bio-electric system (energy flow) (Figure 8) in which 60% of the calorific value of the biomass is transformed into electric energy through a heat engine with 36% efficiency. The thermal efficiency of the engine is 50%. 40% of the calorific value of the biomass generates a certain amount of steam which allows producing electric energy with 25% efficiency through a steam turbine. Part of the electric and thermal energy generated, and the combustion fumes are used by the bioelectromagnetic accelerator.

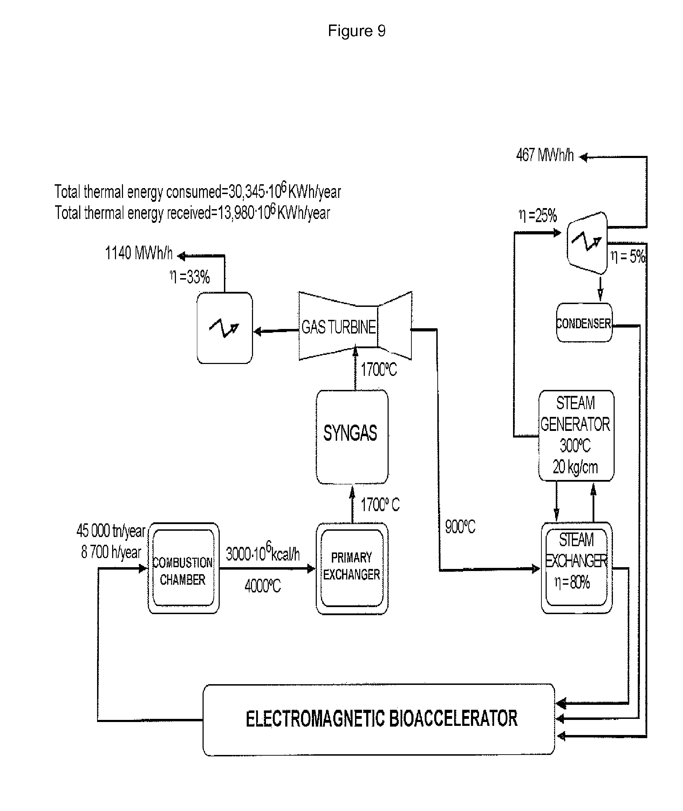

It would also operate like a 100h combined cycle bio-electric system (Figure 9) in which the calorific value of the biomass contained in a fuel gas called syngas (CO₂ and NOx) is used by a gas turbine to generate electric energy. The exhaust gases allow generating steam which also produces electric energy through s steam turbine.

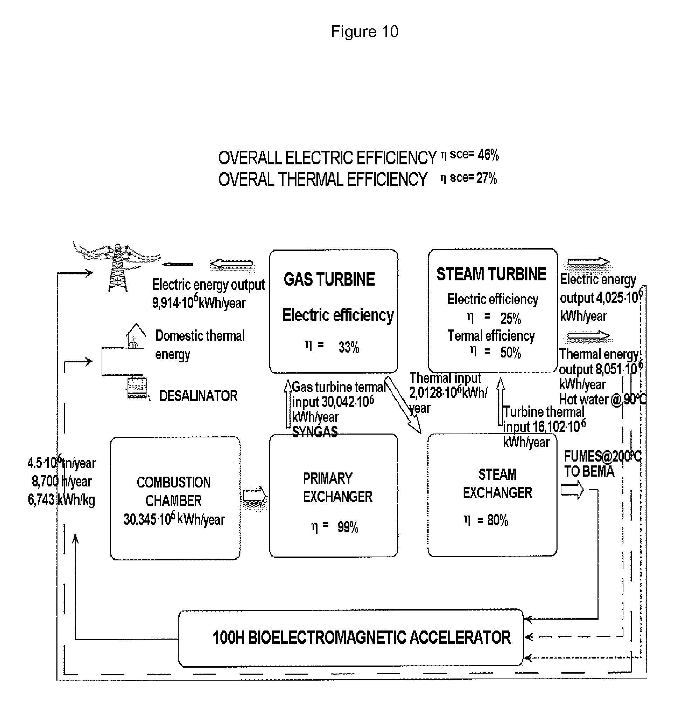

Finally, it could also operate like a 100h combined cycle bio-electric system (energy flow) (Figure 10) in which the calorific value of the biomass is contained in a fuel gas called syngas (CO₂ and NOx) which is obtained by means of plasma gasification. This gas allows generating electric energy through a gas turbine with 33% efficiency. The exhaust gases of the turbine supply heat to a steam generator which will produce electric energy with 25% efficiency through a steam turbine. Part of the electric and thermal energy generated and the combustion fumes are used by the bioelectromagnetic accelerator.

### Brief Description of the Drawings

Figure 1 shows a representative diagram of the accelerated process for the energy conversion of carbon dioxide object of the present invention with each of its steps for the use of solar and artificial electromagnetic energy for the purpose of obtaining, among others, products the oxidation of which generates a total use of the thermal energy produced by all carbon compounds after their oxidation.
Figure 2 shows a representative diagram of the accelerated process for the energy conversion of carbon dioxide object of the present invention in which an additional step has been added between step 2 and step 3. In this new step, a transformation process is carried out on the products obtained in the second step. The lipids are aimed at a chemical energy transformation process by means of transesterification. The hydrocarbons are distilled by means of catalytic hydrocracking, thus obtaining energy products such as kerosene, benzene, biodiesel, naphthas and others such as glycerin. A molecular breakup is applied to the sugars to obtain ethanol, part of which will be used in the transesterification process carried out in the lipids.
Figure 3 shows a representative diagram of the process for the recovery of the carbon dioxide emitted by car engines, which is captured at the exhaust pipe outlet in normal engine operating conditions. Then the gases are compressed and accumulated in a deposit independent from the vehicle, similar to a fuel deposit or tank. Then this deposit is emptied in service stations at the same time the vehicle is filled up with fuel. These carbon dioxide collection tanks or deposit are then reinjected in the electromagnetic bioaccelerators of an accelerated type energy production plant for the energy conversion of carbon dioxide while at the same time said plant produces the necessary fuel for the vehicle.
Figure 4 shows a representative diagram of a possible application of the process described in the present invention, such that for example it would be a constituent part of an incineration plant and therefore assure a continuous source of carbon dioxide as a supply of nutrients to the electromagnetic bioaccelerators. The figure also shows a representative diagram of the electromagnetic bioaccelerator in which the culture of the phytoplankton is carried out and of the steps that are followed for the production of biofuels, CO₂, NOx...

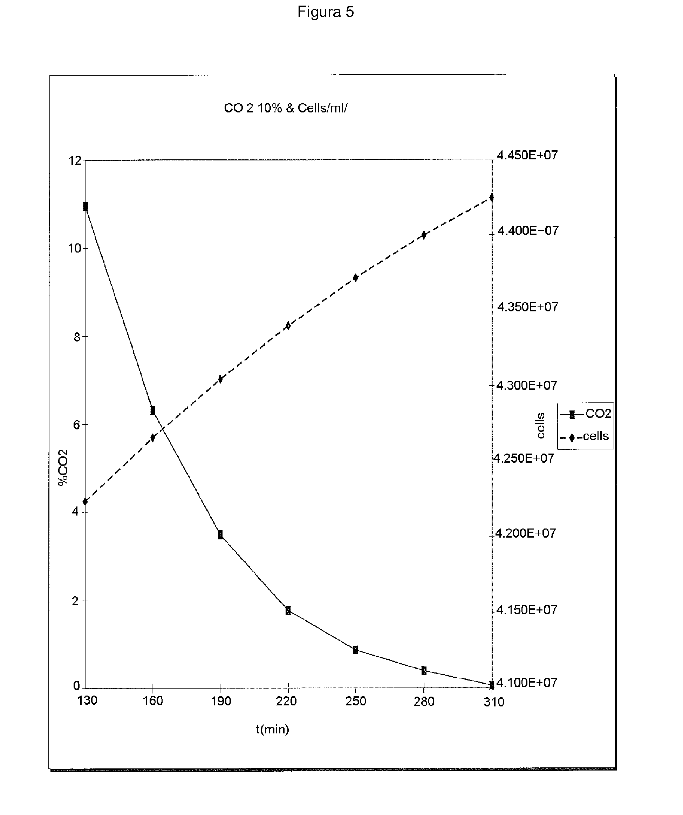

Figure 5 shows the attenuation of atmospheric CO₂ at a concentration of 10% v/v by means of the use of the *Nannochloropsis gaditana* strain.

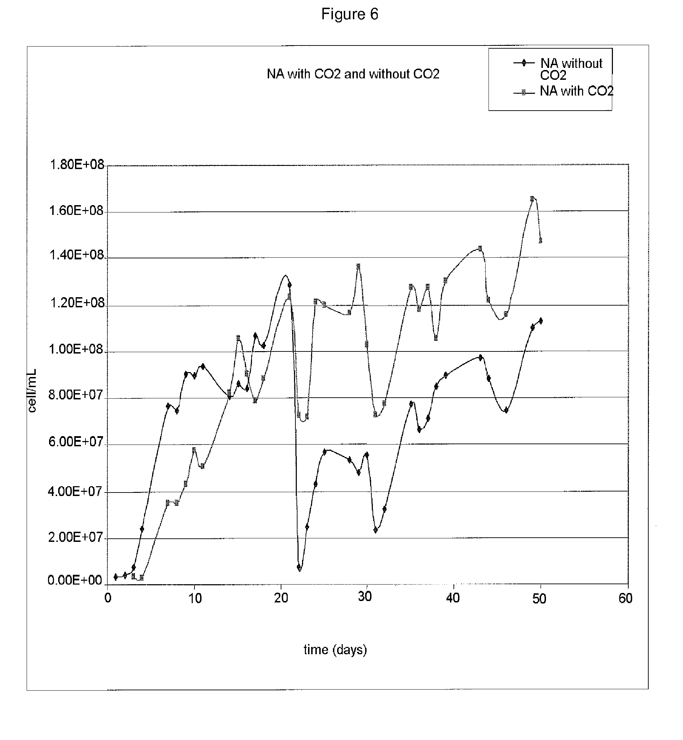

Figure 6 shows the effect of CO₂ on the increase of biomass in a culture of a *Nannochloropsis sp* type strain in which NA represents said type strain.
Figure 7 shows a 1h heat engine cycle bio-electric system in which the calorific value of the biomass is used by a heat engine to generate electric energy and by a steam exchanger; this steam also allows producing electric energy through a steam turbine.
Figure 8 shows a 1h heat engine cycle bio-electric system (energy flow) in which 60% of the calorific value of the biomass is transformed into electric energy through a heat engine with 36% efficiency. The thermal efficiency of the engine is 50%. 40% of the calorific value of the biomass generates a certain amount of steam that allows producing electric energy with 25% efficiency through a steam turbine. Part of the electric and thermal energy generated and the combustion fumes are used by the bioelectromagnetic accelerator.
Figure 9 shows a 100h combined cycle bio-electric system in which the calorific value of the biomass contained in a fuel gas called syngas (CO₂ and NOx) is used by a gas turbine to generate electric energy. The exhaust gases allow generating steam which also produces electric energy through a steam turbine.
Figure 10 shows a 100h combined cycle bio-electric system (energy flow) in which the calorific value of the biomass is contained in a fuel gas called syngas (CO₂ and NOx) which is obtained by means of plasma gasification. This gas allows generating electric energy through a gas turbine with 33% efficiency. The exhaust gases of the turbine supply heat to a steam generator, which will produce electric energy with 25% efficiency through a steam turbine. Part of the electric and thermal energy generated and the combustion fumes are used by the bioelectromagnetic accelerator.

### Embodiment

Figure 5 shows that by using a culture of 41 million cells/ml in a time interval of 310 minutes, a reduction in an atmosphere rich in CO₂ at 10% of all the CO₂ existing in said atmosphere was obtained, with a biomass increase of 3.5 million cells/ml. The culture was maintained stable at 22°C and pH was maintained constant at 8.2. Light was maintained in an 18:6 photoperiod. Experiments conducted in enriched atmospheres at 20% show a similar pattern and direct proportionality to the biomass increase. The species used was *Nannochloropsis gaditana*. The salinity of the medium was 38 per thousand and the experiment was conducted in a closed culture fermenter with a volume of 40 liters.

The initial strains for the biomass converter inoculation are maintained in microfiltered seawater using 0.45 micron cellulose acetate filters and subsequent 0.20 micron re-filtering, and finally sterilized using UV rays. The culture medium of the converters is kept sterile and axenic by means of antibiotics and fungicides.

The antibiotics added to the culture are a mixture of penicillin and streptomycin in a range of concentrations from 100 to 300 mg/I each, preferably in a range of concentrations from 150 to 250 mg/l and more preferably at a concentration of 200 mg/l for each of the components of the mixture.

The fungicides added to the culture are a mixture of griseofulvin and nystatin in a range of concentrations from 100 to 300 mg/l each, preferably in a range of concentrations from 150 to 250 mg/l and more preferably at a concentration of 200 mg/l for each of the components of the mixture.

Figure 6 shows the difference in the growth of two *Nannochloropsis sp* cultures, the only difference being the presence or absence of air enriched with CO₂ at 5%. As can be seen in the figure, growth of the strain with atmospheric air is in the order of 40% less than the growth of the strain cultured with air enriched with CO₂ at 5%. This experiment was conducted in a 0.5 m³ electromagnetic bioaccelerator under temperature, salinity and pH conditions identical to the previous case.

The difference in the efficiency of the strain in the presence and of the strain in the absence of air enriched with in CO₂ at 5% becomes especially important once the 120 million cells/ml have been exceeded.

The initial strains for the biomass converter inoculation are maintained in microfiltered seawater using 0.45 micron cellulose acetate filters and subsequent 0.20 micron re-filtering, and finally sterilized using UV rays. The culture medium of the converters is kept sterile and axenic by means of antibiotics and fungicides.

The antibiotics added to the culture are a mixture of penicillin and streptomycin in a range of concentrations from 100 to 300 mg/l each, preferably in a range of concentrations from 150 to 250 mg/l and more preferably at a concentration of 200 mg/l for each of the components of the mixture.

The fungicides added to the culture are a mixture of griseofulvin and nystatin in a range of concentrations from 100 to 300 mg/l each, preferably in a range of concentrations from 150 to 250 mg/l and more preferably at a concentration of 200 mg/l for each of the components of the mixture.

## Claims

1. A process for the energy conversion of carbon dioxide, **characterized in that** it comprises the following steps:
a. culturing phytoplankton in electromagnetic bioaccelerators;
b. producing oxygen and biomass made up of lipids, hydrocarbons and sugars from the previous step;
c. oxidizing the hydrocarbons produced in the previous step to generate carbon dioxide and NOx; and
d. collecting the carbon dioxide and NOx from the previous step until the cultures of the first step.

2. A process for the energy conversion of carbon dioxide according to claim 1, **characterized in that** the following culture conditions occur in step a:
a. constant temperature within the range of 20 to 25°C;
b. wavelengths within the range of 400 to 700 nm;
c. solar light intensity from 200 to 900 watts/m²;
d. artificial light intensity from 1 to 50 watts/m²;
e. photoperiods from 24:0 to 12:12 light/dark hours;
f. salinity from 0.2‰ to 40‰, preferably from 20‰ to 40‰ for salt water strains, 8‰ to 20‰ for brackish water strains and 0.2‰ to 8‰ for fresh water strains;
g. pressure from 1 to 5 atmospheres;
h. antibiotics and fungicides at a concentration from 100 to 300 mg/ml, preferably from 150 to 250 mg/ml and more preferably at 200 mg/ml
i. concentration of phytoplankton or zooplankton from 30 to 500 million cells/ml; and
j. pH from 6.5 to 8.9.

3. A process for the energy conversion of carbon dioxide according to claims 1 to 2, **characterized in that** in step a, the culture of phytoplankton is subjected to an electric field and to a magnetic field.

4. A process for the energy conversion of carbon dioxide according to claim 1, **characterized in that** step b comprises the following steps:
a. extracting the biomass from the culture medium;
b. centrifuging the biomass;
c. drying the biomass;
d. separating silicates and cellulose by means of apolar solvents; and
e. breaking up the cells of the culture medium by means of ultrasounds, polytron, microwaves and/or heating at 200°C.

5. A process for the energy conversion of carbon dioxide according to claim 1, **characterized in that** in step c the hydrocarbons are oxidized by means of direct and/or indirect combustion.

6. A process for the energy conversion of carbon dioxide according to claim 1, **characterized in that** the gases from step c are collected in step d to be taken back to the culture medium of step a.

7. The use of the NOx and of the carbon dioxide generated in step c of the process of claim 1 to produce steam for its use in turbines.

8. The use of the NOx and of the carbon dioxide generated in step c of the process of claim 1 to feed the phytoplankton of the culture medium.

9. The use of the NOx and of the carbon dioxide generated in step c of the process of claim 1 to produce steam for its use in Stirling type engines.

10. The use of the NOx and of the carbon dioxide generated in step c of the process of claim 1 to produce steam for its use in Stirling-turbine combined cycles.

11. A process for the energy conversion of carbon dioxide according to claims 1 to 6, **characterized in that** a step can additionally be incorporated between steps b and c in which a transformation of the products resulting from step b into high energy level compounds occurs.

12. A process for the energy conversion of carbon dioxide according to claim 11, **characterized in that** in step e the lipids from step b undergo a transesterification process.

13. A process for the energy conversion of carbon dioxide according to claim 11, **characterized in that** in step e the hydrocarbons from step b are distilled by means of catalytic hydrocracking to obtain energy products such as kerosene, benzene, biodiesel, naphthas and glycerin.

14. A process for the energy conversion of carbon dioxide according to claim 11, **characterized in that** in step e the sugars from step b undergo a molecular breakup process to obtain ethanol.
